(19) Europäisches Patentamt
European Patent Office
Office européen des brevets

(11) EP 2 828 268 B1

(12) # EUROPÄISCHE PATENTSCHRIFT

(45) Veröffentlichungstag und Bekanntmachung des Hinweises auf die Patenterteilung:
**14.12.2016 Patentblatt 2016/50**

(21) Anmeldenummer: **13713113.2**

(22) Anmeldetag: **18.03.2013**

(51) Int Cl.:
***C07D 495/14*** *(2006.01)*

(86) Internationale Anmeldenummer:
**PCT/EP2013/055565**

(87) Internationale Veröffentlichungsnummer:
**WO 2013/139736 (26.09.2013 Gazette 2013/39)**

(54) **VERFAHREN ZUR HERSTELLUNG VON DITHIIN-TETRACARBOXIMIDEN**

METHOD FOR PRODUCING DITHIIN TETRACARBOXIMIDES

PROCÉDÉ DE FABRICATION DE DITHIINE-TÉTRACARBOXIMIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.03.2012 EP 12161009**

(43) Veröffentlichungstag der Anmeldung:
**28.01.2015 Patentblatt 2015/05**

(73) Patentinhaber:
- **Bayer CropScience AG
40789 Monheim am Rhein (DE)**
- **Bayer Intellectual Property GmbH
40789 Monheim am Rhein (DE)**

(72) Erfinder:
- **HIMMLER, Thomas
51519 Odenthal (DE)**
- **GELLER, Thomas
51519 Odenthal (DE)**
- **RODEFELD, Lars
51375 Leverkusen (DE)**
- **FORD, Mark James
61389 Schmitten (DE)**
- **HÖMBERGER, Günter
65817 Eppstein (DE)**
- **HEINZ, Dieter
51467 Bergisch Gladbach (DE)**

(74) Vertreter: **Bosser, Isabelle
Bayer S.A.S.
Patents & Licensing Department
14 impasse Pierre Baizet
CS99163
69263 Lyon Cedex 09 (FR)**

(56) Entgegenhaltungen:
**WO-A1-2011/128263 US-A- 3 364 229**

- **VALLA ALAIN ET AL: "Atypical oxidation reaction by thionyl chloride. Easy two-step synthesis of N-alkyl-1,4-dithiines", SYNTHETIC COMMUNICATIONS, TAYLOR & FRANCIS GROUP, PHILADELPHIA, PA, Bd. 36, Nr. 23, 1. November 2006 (2006-11-01), Seiten 3591-3597, XP002599895, ISSN: 0039-7911, DOI: 10.1080/00397910600943600**
- **ZENTZ F ET AL: "Syntheses, in vitro antibacterial and antifungal activities of a series of N-alkyl, 1,4-dithiines", IL FARMACO, ELSEVIER FRANCE * EDITIONS SCIENTIFIQUES ET MEDICALES, IT, Bd. 60, Nr. 11-12, 1. November 2005 (2005-11-01), Seiten 944-947, XP027697616, ISSN: 0014-827X [gefunden am 2005-11-01]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 828 268 B1

Printed by Jouve, 75001 PARIS (FR)

## Beschreibung

Die vorliegende Erfindung betrifft ein neues Verfahren zur Herstellung von Dithiin-tetracarboximiden durch Umsetzung von Bernsteinsäuremonoamiden mit Thionylchlorid, wobei mindestens ein definierter Verfahrensschritt kontinuierlich geführt wird.

Dithiin-tetracarboximide als solche sind bereits bekannt. Ebenso ist bekannt, dass diese Dithiin-tetracarboximide als Anthelminthika gegen innere Parasiten von Tieren, insbesondere Nematoden, verwendet werden können und insektizide Wirkung aufweisen (vgl. US 3,364,229). Außerdem ist bekannt, dass bestimmte Dithiin-tetracarboximide antibakterielle Wirkung besitzen und gegen Erreger von Mykosen beim Menschen eine gewisse Wirkung aufweisen (vgl. Il Farmaco 2005, 60, 944-947). Weiterhin ist bekannt, dass Dithiin-tetracarboximide als Pigmente in elektrophotographischen Photorezeptoren oder als Farbstoffe in Lacken und Polymeren eingesetzt werden können (vgl. JP-A 10-251265, PL-B 143804). Auch deren Verwendung als Fungizid ist bekannt (WO 2011/128263 A1).

Dithiin-tetracarboximide der Formel (I)

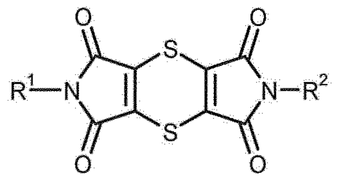

(I)

in welcher

$R^1$ und $R^2$ gleich oder verschieden sind und für Wasserstoff, für gegebenenfalls einfach oder mehrfach durch Halogen, $-OR^3$, $-COR^4$ substituiertes $C_1$-$C_8$-Alkyl, gegebenenfalls einfach oder mehrfach durch Halogen, $C_1$-$C_4$-Alkyl oder $C_1$-$C_4$-Halogenalkyl substituiertes $C_3$-$C_7$-Cycloalkyl, jeweils gegebenenfalls einfach oder mehrfach durch Halogen, $C_1$-$C_4$-Alkyl, $C_1$-$C_4$-Halogenalkyl, $-COR^4$ oder Sulfonylamino substituiertes Aryl oder Aryl-($C_1$-$C_4$-alkyl) stehen,

$R^3$ für Wasserstoff, $C_1$-$C_4$-Alkyl, $C_1$-$C_4$-Alkylcarbonyl oder für gegebenenfalls einfach oder mehrfach durch Halogen, $C_1$-$C_4$-Alkyl oder $C_1$-$C_4$-Halogenalkyl substituiertes Aryl steht,

$R^4$ für Hydroxy, $C_1$-$C_4$-Alkyl oder $C_1$-$C_4$-Alkoxy steht,

können auf verschiedene bekannte Weisen hergestellt werden.

Beispielsweise wird in einem Verfahren (vgl. US 3,364,229; Chem. Ber. 1967, 100, 1559-1570) in einer ersten Stufe Dichlormaleinsäureanhydrid der Formel (II) mit einem Amin der Formel (III) gegebenenfalls in Gegenwart eines Verdünndungsmittels umgesetzt. Anschließend werden dann die so erhaltenen Dichlormaleinsäureimide der Formel (IV) mit einer Schwefelverbindung (z.B. Schwefelwasserstoff oder Thioharnstoff) umgesetzt. Die Herstellung der Dithiin-tetracarboximide der Formel (I) nach diesem Verfahren kann durch das folgende Schema veranschaulicht werden:

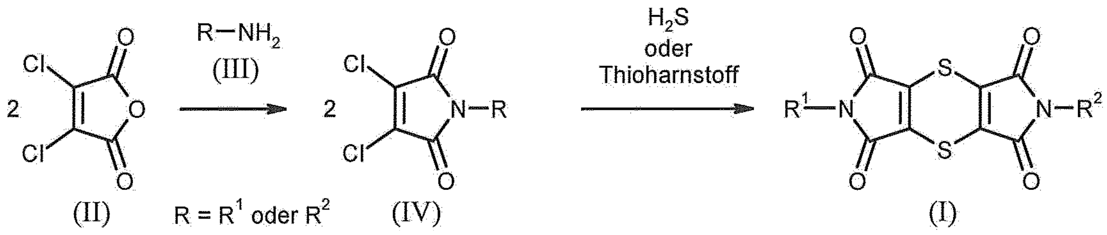


(II) (IV) (I)

Dieses Verfahren hat den Nachteil, dass beispielsweise der Umgang mit dem hochgiftigen Schwefelwasserstoffgas technisch sehr schwierig und aufwendig ist. Bei der Verwendung von Thioharnstoff werden außer dem Zielprodukt unerwünschte Nebenprodukte erhalten, die nur sehr schwierig zu entfernen sind und die erreichbaren Ausbeuten verschlechtern (vgl. J. Heterocycl. Chem. 1988, 25, 901-906).

In einem weiteren bekannt gewordenen Verfahren (vgl. Synthetic Communications 2006, 36, 3591-3597) wird in einer ersten Stufe Bernsteinsäureanhydrid der Formel (V) mit einem Amin der Formel (III) gegebenenfalls in Gegenwart eines Verdünndungsmittels umgesetzt. Anschließend werden die so erhaltenen Bernsteinsäuremonoamide der Formel (VI) 6 Stunden mit einem großen Überschuss an Thionylchlorid in Gegenwart von Dioxan als Verdünnungsmittel bei Raumtemperatur umgesetzt, wobei in einer Abfolge zahlreicher Reaktionsschritte schließlich die Dithiin-tetracarboximide der Formel (I) erhalten werden. Die Dithiin-tetracarboximide werden wahlweise direkt aus dem Reaktionsgemisch oder

nach Versetzen mit Wasser durch Filtration isoliert. Je nach Reaktionsbedingungen (Verdünnungsmittel) und Art der Reste R können unter Umständen die Dithiin-diisoimide der Formel (VII) isoliert werden, bevor man sie in die Dithiin-tetracarboximide der Formel (I) überführt. Diese Herstellungsmethode der Dithiin-tetracarboximide der Formel (I) kann durch das folgende Schema veranschaulicht werden:

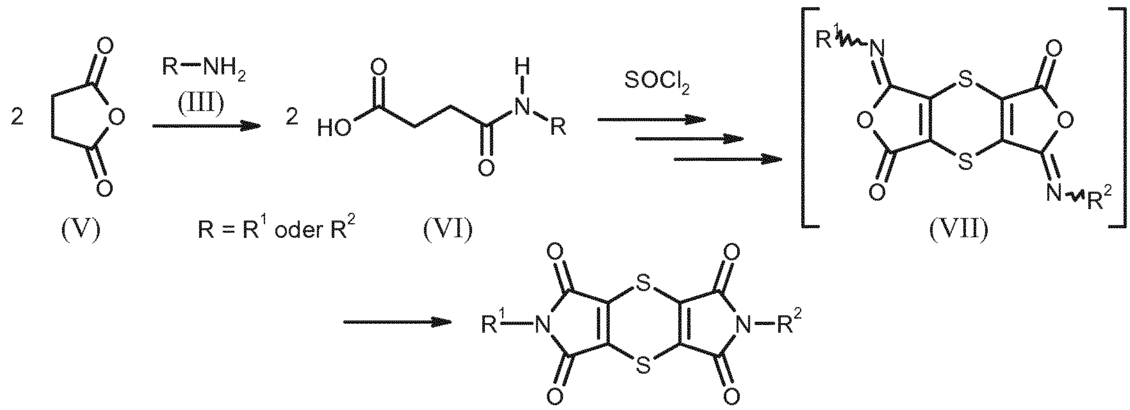

(V) (VI) (VII)

(I)

Nachteilig an diesem Verfahren sind die lange Reaktionszeit sowie das Ergebnis, dass entweder die erhaltenen Ausbeuten in der Regel etwa 30-40% der Theorie nicht überschreiten oder aber die Reinheiten der isolierten Produkte ungenügend sind. Außerdem ist bei einer wässrigen Aufarbeitung des Reaktionsgemisches nachteilig, dass dabei große Mengen Thionylchlorid vernichtet werden; die entstehenden Gase ($SO_2$ und HCl) müssen entsorgt werden. Ebenfalls nachteilig ist der Umstand, dass erfahrungsgemäß das Produkt nicht in einer Fraktion erhalten wird. Vielmehr ist es häufig so, dass nach einer ersten Produktisolierung durch Filtration aus dem Filtrat nach längerem Stehen (beispielsweise über Nacht), weiteres Produkt ausfällt, das erneut durch Filtration isoliert werden muss. Zuweilen muss dieser Vorgang nochmals durchgeführt werden. Diese Arbeitsweise ist sehr umständlich und zeitraubend.

Weiterhin ist bekannt, dass Dithiin-tetracarboximide erhalten werden, indem N-substituierte Bernsteinsäureamide in trockenem 1,4-Dioxan gelöst und anschließend mit Thionylchlorid versetzt werden. Anschließend wird das Reaktionsgemisch erwärmt und die Lösung wird in vacuo eingeengt und über Säulenchromatographie aufgetrennt und gereinigt (vgl. J. Heterocycl. Chem. 2010, 47, 188-193).

Weiterhin ist bekannt, dass man Dithiin-tetracarboximide erhält, indem man N-substituiene Bernsteinsäureamide gegebenenfalls in Gegenwart eines inerten Verdünnungsmittels mit Thionylchlorid versetzt. Anschließend wird das überschüssige Thionylchlorid abdestilliert, das zurückbleibende Reaktionsgemisch in Gegenwart von Wasser und gegebenenfalls in Gegenwart eines inerten Verdünnungsmittels erwärmt (vgl. WO 2011/128263).

Die Verfahren des Standes der Technik weisen jedoch folgende Nachteile auf:

a) die geringe Raum-Zeit-Ausbeute,

b) der große Überschuss an Thionylchlorid bezogen auf die Bernsteinsäuremonoamide, der einen hohen technischen Aufwand bei der Aufarbeitung des Reaktionsaustrags und Rückgewinnung des unumgesetzten Thionychlorids und damit hohe Investions- und Energiekosten bedingt,

c) der bei der Reaktion freiwerdende Abgasstrom verläuft nicht gleichmäßig und damit ist ein technisch einfaches und ökonomisches Herstellverfahren, das gegebenenfalls die gleichzeitige Wiederaufarbeitung des Abgasstromes zum Zwecke der Wiederverwertung beinhaltet, erschwert.

Aufgabe der vorliegenden Erfindung bestand somit darin, ein technisch einfaches und ökonomisches Herstellverfahren für Dithiin-tetracarboximide der Formel (I) in hohen Ausbeuten und Raum-Zeit-Ausbeuten und hoher Qualität bereitzustellen.

Es wurde überraschenderweise gefunden, dass die Verbindungen der Formel (I),

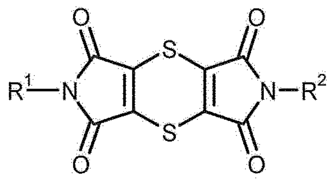

(I)

in welcher

| | |
|---|---|
| $R^1$ und $R^2$ | gleich oder verschieden sind und für Wasserstoff, für gegebenenfalls einfach oder mehrfach durch Halogen, $-OR^3$, $-COR^4$ substituiertes $C_1$-$C_8$-Alkyl, gegebenenfalls einfach oder mehrfach durch Halogen, $C_1$-$C_4$-Alkyl oder $C_1$-$C_4$-Halogenalkyl substituiertes $C_3$-$C_7$-Cycloalkyl, jeweils gegebenenfalls einfach oder mehrfach durch Halogen, $C_1$-$C_4$-Alkyl, $C_1$-$C_4$-Halogenalkyl, $-COR^4$ oder Sulfonylamino substituiertes Aryl oder Aryl-($C_1$-$C_4$-alkyl) stehen, |
| $R^3$ | für Wasserstoff, $C_1$-$C_4$-Alkyl, $C_1$-$C_4$-Alkylcarbonyl oder für gegebenenfalls einfach oder mehrfach durch Halogen, $C_1$-$C_4$-Alkyl oder $C_1$-$C_4$-Halogenalkyl substituiertes Aryl steht, |
| $R^4$ | für Hydroxy, $C_1$-$C_4$-Alkyl oder $C_1$-$C_4$-Alkoxy steht, |

sowohl in hoher Ausbeute als auch in hoher Raum-Zeit-Ausbeute erhalten werden, wenn mindestens ein definierter Verfahrensschritt kontinuierlich geführt wird.

Vor- und nachstehend bedeutet der Begriff "kontinuierlich" eine Verfahrensweise, bei der sich Reaktionsmedien in einem Durchfluss-System befinden, und durch bestimmte Funktionszonen, insbesondere Mischzonen, Reaktionszonen und Verweilzonen geleitet werden. Dabei werden die Edukte in definierten Zeiteinheiten zugeführt und die Produkte in definierten Zeiteinheiten abgeführt.

Vor- und nachstehend bedeutet der Begriff "Edukt" eine chemische Verbindung, die in einem nachfolgenden Reaktionsschritt weiterverarbeitet wird. Dieser Begriff umfasst sowohl chemische Verbindungen, die neu in den Gesamtprozess eingeführt werden als auch solche, die in einem vorgeschalteten Prozess erzeugt wurden und weiterverarbeitet werden.

Gegenstand der vorliegenden Erfindung ist ein Verfahren zur Herstellung von Dithiin-tetracarboximide der allgemeinen Formel (I)

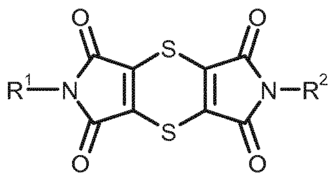

(I)

in welcher

| | |
|---|---|
| $R^1$ und $R^2$ | gleich oder verschieden sind und für Wasserstoff, für gegebenenfalls einfach oder mehrfach durch Halogen, $-OR^3$, $-COR^4$ substituiertes $C_1$-$C_8$-Alkyl, gegebenenfalls einfach oder mehrfach durch Halogen, $C_1$-$C_4$-Alkyl oder $C_1$-$C_4$-Halogenalkyl substituiertes $C_3$-$C_7$-Cycloalkyl, jeweils gegebenenfalls einfach oder mehrfach durch Halogen, $C_1$-$C_4$-Alkyl, $C_1$-$C_4$-Halogenalkyl, $-COR^4$ oder Sulfonylamino substituiertes Aryl oder Aryl-($C_1$-$C_4$-alkyl) stehen, |
| $R^3$ | für Wasserstoff, $C_1$-$C_4$-Alkyl, $C_1$-$C_4$-Alkylcarbonyl oder für gegebenenfalls einfach oder mehrfach durch Halogen, $C_1$-$C_4$-Alkyl oder $C_1$-$C_4$-Halogenalkyl substituiertes Aryl steht, |
| $R^4$ | für Hydroxy, $C_1$-$C_4$-Alkyl oder $C_1$-$C_4$-Alkoxy steht, |

dadurch gekennzeichnet, dass in einer ersten Stufe (1) Bernsteinsäuremonoamide der Formel (VI)

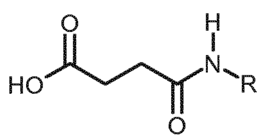

(VI)

(VI)

in welcher R für $R^1$ oder $R^2$ steht,
mit Thionylchlorid zu einer Reaktionsmischung umgesetzt werden, in einer zweiten Stufe (2) die entstandene Reaktionsmischung erhitzt wird und anschließend die Umsetzung der Reaktionsmischung zu den Dithiin-tetracarboximiden im dritten Schritt (3) erfolgt, wobei mindestens Schritt (2) kontinuierlich durchgeführt wird.

Bevorzugt beträgt im erfindungsgemäßen Verfahren die Summe an Thionylchlorid (z) zwischen 2,5 und 20 Mol pro Mol Bernsteinsäuremonoamid der Formel (VI), wobei z durch die Beziehung

$$z = x + y$$

definiert wird und

- z für die Gesamtmenge an Thionylchlorid (Mol Thionylchlorid pro Mol Bernsteinsäureamid der allgemeinen Formel (VI)) in den beiden ersten Schritten (1) und (2),
- x für die Menge Thionylchlorid in Schritt (1) und
- y für die in Schritt (2) zusätzlich eingesetzte Menge Thionylchlorid steht.

Im erfindungsgemäßen Verfahren liegt besonders bevorzugt der Wert für z zwischen 2,5 und 14 und ganz besonders bevorzugt zwischen 2,5 und 9.

Wird die Gesamtmenge an Thionylchlorid (z) aufgeteilt in Schritt (1) und (2) des erfindungsgemäßen Verfahrens zugegeben, gelten die folgenden Angaben:

Der Wert für x liegt zwischen 1 und 20, bevorzugt zwischen 1 und 10, besonders bevorzugt zwischen 1 und 5, wobei die oben genannten Werte für z gelten.

In der ersten Stufe (1) werden die Bernsteinsäuremonoamide der allgemeinen Formel (VI)

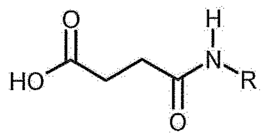

(VI) (VI)

in welcher R für $R^1$ oder $R^2$ steht,
mit Thionylchlorid zu einer flüssigen Reaktionsmischung umgesetzt.

In der zweiten Stufe (2) wird die entstandene Reaktionsmischung, gegebenenfalls mit weiterem Thionylchlorid, erhitzt.

Nach Abklingen der Gasentwicklung erfolgt die Umsetzung der Reaktionsmischung zu den Dithiin-tetracarboximiden im dritten Schritt (3) des Verfahrens. In diesem wird, gegebenenfalls nach Entfernung von überschüssigem Thionylchlorid, das erhaltene Zwischenprodukt in einem Verdünnungsmittel gelöst, mit Wasser versetzt und durch Erwärmen in diesem Gemisch in die Dithiin-tetracarboximide der Formel (I) überführt.

Auf diese Weise können die Dithiin-tetracarboximide der Formel (I) in einem technisch einfachen und ökonomischen Verfahren erhalten werden, wobei der Überschuss Thionylchlorid abgetrennt wird und der konstante Abgasstrom eine technisch einfache Wiederaufarbeitung der Abgase erlaubt.

Die bei der Durchführung des erfindungsgemäßen Verfahrens als Ausgangstoffe eingesetzten Bernsteinsäuremonoamide sind durch die Formel (VI) allgemein definiert. R steht für die Bedeutungen von $R^1$ oder $R^2$.

In einer Ausführungsform (A-I) der Verbindung der allgemeinen Formel (VI) sind $R^1$ und $R^2$ gleich oder verschieden und stehen für Wasserstoff, für gegebenenfalls einfach oder mehrfach durch Fluor, Chlor, Brom,-$OR^3$, -$COR^4$ substituiertes $C_1$-$C_6$-Alkyl, gegebenenfalls einfach oder mehrfach durch Chlor, Methyl oder Trifluormethyl substituiertes $C_3$-$C_7$-Cycloalkyl, für jeweils gegebenenfalls einfach oder mehrfach durch Fluor, Chlor, Brom, Methyl, Trifluormethyl, -$COR^4$, Sulfonylamino substituiertes Phenyl oder Phenyl-($C_1$-$C_4$-alkyl).

In einer Ausführungsform (A-I-1) der Verbindung der allgemeinen Formel (VI) sind $R^1$ und $R^2$ gleich oder verschieden und stehen für Wasserstoff, für gegebenenfalls einfach oder mehrfach durch Fluor, Chlor, Hydroxy, Methoxy, Ethoxy, Methylcarbonyloxy, Carboxyl substituiertes $C_1$-$C_4$-Alkyl, gegebenenfalls einfach oder mehrfach durch Chlor,

Methyl oder Trifluormethyl substituiertes $C_3$-$C_7$-Cycloalkyl, für jeweils gegebenenfalls einfach bis dreifach durch Fluor, Chlor, Brom, Methyl, Trifluormethyl, $-COR^4$, Sulfonylamino substituiertes Phenyl, Benzyl, 1-Phenethyl, 2-Phenethyl oder 2-Methyl-2-phenethyl.

In einer Ausführungsform (A-I-2) der Verbindung der allgemeinen Formel (VI) sind $R^1$ und $R^2$ sind gleich oder verschieden und stehen für Wasserstoff, Methyl, Ethyl, n-Propyl, Isopropyl, 2,2-Difluorethyl, 2,2,2-Trifluorethyl, jeweils gegebenenfalls durch Chlor, Methyl oder Trifluormethyl substituiertes Cyclopropyl oder Cyclohexyl.

In einer Ausführungsform (A-I-3) der Verbindung der allgemeinen Formel (VI) stehen $R^1$ und $R^2$ gleichzeitig für Methyl.

Ausführungsform (B-I): Verbindung der allgemeinen Formel (VI) entsprechend der Ausführungsformen (A-I) oder (A-I-1) oder (A-I-2), in welcher $R^3$ für Wasserstoff, Methyl, Ethyl, Methylcarbonyl, Ethylcarbonyl oder für gegebenenfalls einfach oder mehrfach durch Fluor, Chlor, Methyl, Ethyl, n-Propyl, Isopropyl oder Trifluormethyl substituiertes Phenyl steht.

Ausführungsform (B-I-1): Verbindung der allgemeinen Formel (VI) entsprechend der Ausführungsformen (A-I) oder (A-I-1) oder (A-I-2), in welcher $R^3$ für Wasserstoff, Methyl, Methylcarbonyl oder für Phenyl steht.

Ausführungsform (C-I): Verbindung der allgemeinen Formel (VI) entsprechend der Ausführungsformen [(A-I) oder (A-I-1) oder (A-I-2)] und/oder [(B-I) oder B(B-I-1)], in welcher $R^4$ für Hydroxy, Methyl, Ethyl, Methoxy oder Ethoxy steht.

Ausführungsform (C-I-1): Verbindung der allgemeinen Formel (VI) entsprechend der Ausführungsformen [(A-I) oder (A-I-1) oder (A-I-2)] und/oder [(B-I) oder B(B-I-1)], in welcher $R^4$ für Hydroxy oder Methoxy steht.

Besonders bevorzugt wird Bernsteinsäuremethylamid als Ausgangsstoff eingesetzt, wodurch man als Endprodukt die Verbindung (I-1) 2,6-Dimethyl-1H,5H-[1,4]dithiino[2,3-c:5,6-c']dipyrrol-1,3,5,7(2H,6H)-tetron erhält.

Wird Bernsteinsäuretertiärbutylamid als Ausgangsstoff eingesetzt, wird die Verbindung (1-2) 2,6-Di-tert-butyl-1H,5H-[1,4]dithiino[2,3-c:5,6-c']dipyrrol-1,3,5,7(2H,6H)-tetron als Endprodukt erhalten.

Wird Bemsteinsäurecyclohexylamid als Ausgangsstoff eingesetzt, wird die Verbindung (I-3) 2,6-Dicyclohexyl-1H,5H-[1,4]dithiino[2,3-c:5,6-c']dipynol-1,3,5,7(2H,6H)-tetron als Endprodukt erhalten.

Wird Bernsteinsäurepropylamid als Ausgangsstoff eingesetzt, wird die Verbindung (I-4) 2,6-Dipropyl-1H,5H-[1,4]dithiino[2,3-c:5,6-c']dipyrrol-1,3,5,7(2H,6H)-tetron als Endprodukt erhalten.

Schritt (1) des erfindungsgemäßen Verfahrens wird kontinuierlich oder batchweise durchgeführt. bevorzugt wird Schritt (1) kontinuierlich durchgeführt.

Die Reaktionstemperatur im ersten Schritt (1) des erfindungsgemäßen Verfahrens kann in weiten Grenzen variiert werden und liegt zwischen -20°C und 50°C, bevorzugt bei Temperaturen von -5°C bis 30°C; besonders bevorzugt von -5°C bis 10°C.

Schritt (2) des erfindungsgemäßen Verfahrens wird kontinuierlich durchgeführt.

Für die kontinuierliche Durführung der Verfahrensschritte des erfindungsgemäßen Verfahrens eignen sich die verschiedenen, dem Fachmann geläufigen, kontinuierlichen Apparate. Beispiele für derartige kontinuierlich betriebene Apparate sind:

a) kontinuierlich betriebene Rührkessel, gegebenenfalls nacheinander geschaltet zu einer Rührkesselkaskade,

b) Rohrreaktor,

c) Dünnfilmverdampfer,

d) Reaktivrektifikator,

e) Mikroreaktor,

f) Kreuzstromreaktor,

g) Umlauf- oder Loop-reaktoren,

oder Kombinationen aus verschiedenen Kontireaktoren.

Bevorzugt ist die Verwendung einer Rührkesselkaskade, Rohrreaktor oder Dünnfilmverdampfer, besonders bevorzugt ist die Verwendung einer Rührkesselkaskade.

Die Reaktionstemperatur im zweiten Schritt (2) des erfindungsgemäßen Verfahrens kann in weiten Grenzen variiert werden und liegt zwischen 0°C und 150°C, bevorzugt bei Temperaturen von 20°C bis 120°C; besonders bevorzugt von 30°C bis 100°C.

Die Verweilzeit im zweiten Schritt (2) des erfindungsgemäßen Verfahrens liegt zwischen 1 Minute und 24

Stunden. Bevorzugt liegt die Verweilzeit zwischen 15 Minuten und 10 Stunden, besonders bevorzugt zwischen 30 Minuten und 6 Stunden.

Der erste Schritt (1) und der zweite Schritt (2) des erfindungsgemäßen Verfahrens kann gegebenenfalls in Gegenwart eines unter den Reaktionsbedingungen möglichst inerten Verdünnungsmittels durchgeführt werden. Als solche Verdünnungsmittel seien beispielhaft aliphatische Kohlenwasserstoffe wie Pentan, Hexan, Heptan, Cyclohexan, Methylcyclohexan, chlorierte Kohlenwasserstoffe wie Methylenchlorid, Chloroform, 1,2-Dichlorethan, aromatische Kohlenwasserstoffe wie Toluol, Xylol, Mesitylen, chlorierte aromatische Kohlenwasserstoffe wie Chlorbenzol, Dichlorbenzol, Ether wie Diethylether, Methyl-tert-butylether, Tetrahydrofuran, Dioxan, Nitrile wie Acetonitril, Propionitril, Butyronitril, Ester wie Essigsäuremethylester und Essigsäureethylester genannt. Bevorzugt sind Methylenchlorid, Chloroform oder 1,2-Dichlorethan, Toluol, Xylol oder Chlorbenzol. Bevorzugt ist es die beiden Verfahrensschritte (1) und (2) ohne Verdünnungsmittel durchzuführen.

Das gegebenenfalls vorhandene Verdünnungsmittel wird bevorzugt ebenfalls unter vermindertem Druck in Schritt (2) des erfindungsgemäßen Verfahrens abdestilliert.

Schritt (3) des erfindungsgemäßen Verfahrens wird kontinuierlich oder batchweise durchgeführt.

Im dritten Schritt (3) des erfindungsgemäßen Verfahrens wird das, gegebenenfalls nach Entfernung des Verdünnungsmittels und/oder überschüssigem Thionylchlorids, erhaltene Zwischenprodukt in einem Verdünnungsmittel gelöst und durch Erwärmen in diesem Lösungsmittel, gegebenenfalls unter Zusatz von Wasser, in die Dithiin-tetracarboximide der Formel (I) überführt. Bevorzugt wird das Reaktionsgemisch hierbei gerührt.

Im dritten Schritt (3) des erfindungsgemäßen Verfahrens wird ein organisches Lösungsmittel oder ein Lösungsmittelgemisch verwendet. Diese Lösungsmittel sind bevorzugt wenigstens teilweise mit Wasser mischbar. Bei schlecht oder nicht mit Wasser mischbaren Lösungsmitteln kann die Mischbarkeit durch ausgewählte Lösungsvermittler erreicht werden (z.B. Phasentransferkatalysator).

Als Verdünnungsmittel für den dritten Schritt (3) des erfindungsgemäßen Verfahrens eignen sich im Einzelnen Wasser, Dimethylsulfoxid, Sulfolan, Alkohole wie beispielsweise Methanol, Ethanol, Propanol, Isopropanol, 1-Butanol, 2-Butanol, Isobutanol, Tertiärbutanol, 1-Pentanol, Cyclopentanol, Cyclohexanol, Ethylenglykol, Ethylenglkyol-monomethylether, Kohlenwasserstoffe wie Hexan, Heptan, Cyclohexan, Methylcyclohexan, Toluol, Xylole, Mesitylen, Chlorbenzol, Dichlorbenzol, Nitrobenzol, Ester wie Essigsäuremethylester, Essigsäureethylester, Amide wie Formamid, N,N-Dimethylformamid; N,N-Dimethylacetamid, N-Methylpyrrolidon, Ether wie Methyl-tert-butylether, Tetrahydrofuran, 1,4-Dioxan, Nitrile wie Acetonitril, Propionitril, Butyronitril, Benzonitril, Ketone wie Aceton, Methyl-ethylketon, Methyl-isobutylketon, Pinakolon, Carbonsäuren wie Ameisensäure, Essigsäure, Propionsäure oder Gemische dieser Verdünnungsmittel.

Bevorzugt sind Wasser, Toluol, Dimethylsulfoxid, Methanol, Ethanol, Propanol, Isopropanol, 1-Butanol, 2-Butanol, Isobutanol, Tertiärbutanol, 1-Pentanol, Cyclohexanol, Ethylenglykol, Essigsäure-methylester, N,N-Dimethylfonnamid; N,N-Dimethylacetamid, Tetrahydrofuran, 1,4-Dioxan, Acetonitril, Aceton, Methyl-ethylketon, Methyl-isobutylketon, Essigsäure oder Gemische dieser Verdünnungsmittel.

Besonders bevorzugt sind Gemische aus Wasser und Toluol, Methanol, Ethanol, Propanol, Isopropanol, 1-Butanol, 2-Butanol, Isobutanol, 1-Pentanol, Essigsäure-methylester, Tetrahydrofuran, 1,4-Dioxan, Acetonitril, Aceton oder Essigsäure.

Das Mischungsverhältnis Wasser zu organischem Lösungsmittel kann dabei in weiten Grenzen von beispielsweise 9 zu 1 bis 1 zu 9 variiert werden.

Die Reaktionstemperatur im dritten Schritt (3) des erfindungsgemäßen Verfahrens kann in weiten Grenzen variiert werden und liegt zwischen 0°C und 180°C bevorzugt bei Temperaturen von 40°C bis 140°C, besonders bevorzugt von 40°C bis 120°C.

Die Reaktionszeit im dritten Schritt (3) des erfindungsgemäßen Verfahrens liegt zwischen 1 Minuten und 24 Stunden, bevorzugt von 15 Minuten bis 12 Stunden, besonders bevorzugt von 1 bis 6 Stunden.

Das erfindungsgemäße Verfahren wird durch folgende Beispiele veranschaulicht, ohne auf sie eingeschränkt zu sein.

**<u>Beispiel 1</u>**

<u>Verwendung eines Dünnschichtverdampfers im zweiten Schritt (2) des erfindungsgemäßen Verfahrens</u>

Eine Reaktionsmischung von 10g [74,5mmol] Bernsteinsäuremethylamid in 74,7g [596,6mmol] Thionylchlorid (Gehalt: 95%) wird innerhalb 2 Stunden auf einen auf 90°C Manteltemperatur aufgeheizten Dünnschichtverdampfer (Durchmesser: 6cm, Länge: 30cm, Wischergeschwindigkeit: 350 UPM) gegeben. Es wird ein konstanter Abgasstrom beobachtet. Es wird im Sumpfgefäß 20g eines braunen Öls und im Destillatgefäß 39,7g Thionylchlorid (entspricht 89,4% des Überschusses) erhalten. Das braune Öl wird zusammen mit 20ml Toluol und 0,15g Aliquat 336 4 Stunden lang am Rückfluss erhitzt. Nach Abkühlung wird der Feststoff abgesaugt, mit Wasser und Ethanol gewaschen und getrocknet.

Es werden 5,8g der Verbindung I (R = Me) erhalten.

**Beispiel 2**

Verwendung einer Füllkörperkolonne im zweiten Schritt (2) des erfindungsgemäßen Verfahrens

Eine Reaktionsmischung von 150g [1,14mol] Bernsteinsäuremethylamid in 243g [2,02mmol] Thionylchlorid (Gehalt: 99%) wird im Kopf einer auf 77°C Manteltemperatur beheizten Füllkörperkolonne (Füllkörper = Raschig-Ringe: 10 mm Länge, 8 mm Breite, 1 mm Wandstärke; Kolonnenlänge: ca. 110 cm, Innendurchmesser: ca. 5 cm) mit aufgesetztem Rückflußkühler in 285 min eindosiert. Im Gegenstrom erfolgt eine Destillation von 412 g Thionylchlorid (Gehalt: 99%), welches in einem Sumpfgefäß am Fuß der Kolonne auf Raumtemperatur temperiert wird. Die Kopftemperatur wird im Temperaturbereich von ca. 50 - 60 °C gehalten. Es wird ein konstanter Abgasstrom beobachtet. Nach Dosierende wird abgekühlt. Es wird im Sumpfgefäß eine schwarze Reaktionsmischung erhalten, welche am Rotationsverdampfer bei ca. 40 mbar und 60 °C aufkonzentriert wird. Das aufkonzentrierte Material wird zusammen mit 190g Toluol und 9g Aliquat 336 4 Stunden lang am Rückfluss erhitzt. Nach Abkühlung wird der Feststoff abgesaugt, mit Wasser und Ethanol gewaschen und getrocknet. Es werden 99 g der Verbindung I (R = Me) erhalten.

**Beispiel 3**

Verwendung einer Rührkesselkaskade im zweiten Schritt (2) des erfindungsgemäßen Verfahrens

Zwei Rührkessel aus Glas werden über einen Schlauch miteinander verbunden, so dass ab einem Füllvolumen von 600ml im ersten Kessel automatisch der Überlauf des Reaktionsgemischs in den zweiten Kessel erfolgt. Der zweite Kessel kann über einen Bodenauslass in ein Vorlagegefäß kontinuierlich und kontrolliert entleert werden.

Für die kontinuierliche Reaktionsführung der Stufe 2 wird zunächst die Stufe 1 separat hergestellt, wobei in 3 Ansätzen jeweils 200 g Bernsteinsäuremethylamid (1,52 mol) mit 322 g Thionylchlorid (Gehalt: 100% 2,71 mol) bei -10°C bis -5°C zur Reaktion gebracht werden. Nach erfolgter Umsetzung wird die flüssige Reaktionsmischung auf Raumtemperatur gebracht.

Zum Anfahren der Reaktionskaskade wird der erste Kessel mit 544 g Thionylchlorid (4,57 mol) beschickt und auf 70°C temperiert. Hierzu wird innerhalb von 5 h ein Ansatz der Stufe 1 (siehe oben) zudosiert, bis die Gasentwicklung beendet ist. Das Gesamtvolumen in Kessel 1 erreicht dabei den Überlauf.

Anschließend werden die beiden anderen Ansätze der Stufe 1 jeweils mit 544g Thionylchlorid (Gehalt: 100 %, 4,57 mol) vermischt und innerhalb von 7,5 h in den Kessel 1dosiert. Dabei läuft das Reaktionsgemisch kontinuierlich in den zweiten Kessel. Sobald im zweiten Kessel ein hold-up von 100 ml erreicht ist, wird aus diesem Kessel kontinuierlich in eine Vorlage abgelassen. Die Innentemperatur im Reaktor 1 beträgt 65-70°C, die in Reaktor 2 80-83°C. Die Verweilzeit im Reaktor 1 beträgt im Mittel 3 h, im Reaktor 2 im Mittel 0,5 h. Simultan wurden alle Abgase beider Reaktoren gemeinsam über eine Gasuhr geführt. Die entstehenden Gasmengen entstehen, nach einer kurzen Initiationsperiode sehr gleichmäßig (siehe Figur 1).

In Figur 1 ist die Gesamtmenge der Abgase in ltr (y-Achse) gegen die dosierte Menge Reaktionsmischung in ml (x-Achse) aufgetragen.

Das aus dem Reaktor 2 abfließende Material wird eingeengt und dem dritten Schritt (3) zur abschließenden Umsetzung zur Verbindung I zugeführt Beispielhaft wurde die Fraktion von 218 bis 311g des aus dem Kessel 2 abfließenden Produkts der Stufe 2 in 114g Toluol vorgelegt und mit 13,1g Aliquat 336 versetzt. Bei 55-60°C ließ man langsam 91g Wasser zugetropfen. Anschließend ließ man 4h bei 80°C rühren. Nach Abkühlen auf Raumtemperatur wurde das feste Produkt auf einer Nutsche abgesaugt und mit 200 ml Wasser und anschließend mit mehreren Portionen Ethanol gewaschen und getrocknet. Es wurden 62,4g dunkel grüner Feststoff erhalten 68% der Theorie.

Nach Abschluß des Versuches wurden aus allen Produktfraktionen nach Aufarbeitung insgesamt 465,7 g Verbindung I (71% der Theorie) erhalten.

**Vergleichsbeispiel 1**

Es werden 5,24 g [40 mmol] Bernsteinsäuremethylamid vorgelegt und bei 15°C 47,6 g [400 mmol] Thionylchlorid zugetropft. Es wird dann auf 80°C erwärmt und 1 Stunde bei dieser Temperatur gerührt. Es tritt eine stärker werdende Gasentwicklung ein, die sich nach einiger Zeit wieder verringert. Das Reaktionsgemisch wird am Rotationsverdampfer eingeengt. Der Rückstand (dunkelbraunes dickes Öl) wird mit 100 ml Methanol/Wasser (1:1) versetzt und 4 Stunden auf 60°C erwärmt. Danach lässt man auf Raumtemperatur abkühlen, saugt den ausgefallenen Feststoff ab und wäscht ihn mit Wasser und Methanol. Nach Trocknen resultieren 4,05 g dunkelgrüner Feststoff, der lt. HPLC-Analyse zu 97,8 Flächen-% aus der Verbindung (I-1) besteht, was einer Ausbeute von 70% der Theorie entspricht.

**Vergleichsbeispiel 2**

Es werden bei 10°C 243,1 g [2,02 mmol] Thionylchlorid (Gehalt: 99%) vorgelegt und 150 g [1,14 mol] Bernsteinsäuremethylamid portionsweise zugegeben. Nach erfolgter Umsetzung wird die gekühlte flüssige Reaktionsmischung in einen Reaktor dosiert, in dem 412 g Thionylchlorid (Gehalt: 99%; 3,42 mol) bei 65 - 70 °C vorgelegt wurden. Bei der Dosierung wird die Temperatur im Bereich von 65 - 70 °C gehalten. Es tritt eine stärker werdende Gasentwicklung ein, die sich nach einiger Zeit wieder verringert. Das Reaktionsgemisch wird am Rotationsverdampfer eingeengt. Der Rückstand (dunkelbraunes dickes Öl) wird mit 190 g Toluol, 161 g Wasser und 9,2 g Aliquat 336 versetzt und ca. 6 Stunden auf 75 - 80 °C erwärmt. Danach lässt man auf Raumtemperatur abkühlen, saugt den ausgefallenen Feststoff ab und wäscht ihn mit Wasser und Ethanol. Nach Trocknen resultieren 118 g dunkelgrüner Feststoff, der lt. HPLC-Analyse zu 98,2 w% aus der Verbindung (I-1) besteht, was einer Ausbeute von 72% der Theorie entspricht.

## Patentansprüche

1. Verfahren zur Herstellung von Dithiin-tetracarboximide der allgemeinen Formel (I)

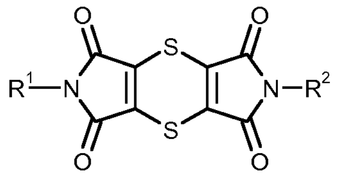

(I)

in welcher

$R^1$ und $R^2$ gleich oder verschieden sind und für Wasserstoff, für gegebenenfalls einfach oder mehrfach durch Halogen, $-OR^3$, $-COR^4$ substituiertes $C_1$-$C_8$-Alkyl, gegebenenfalls einfach oder mehrfach durch Halogen, $C_1$-$C_4$-Alkyl oder $C_1$-$C_4$-Halogenalkyl substituiertes $C_3$-$C_7$-Cycloalkyl, jeweils gegebenenfalls einfach oder mehrfach durch Halogen, $C_1$-$C_4$-Alkyl, $C_1$-$C_4$-Halogenalkyl, $-COR^4$ oder Sulfonylamino substituiertes Aryl oder Aryl-($C_1$-$C_4$-alkyl) stehen,
$R^3$ für Wasserstoff, $C_1$-$C_4$-Alkyl, $C_1$-$C_4$-Alkylcarbonyl oder für gegebenenfalls einfach oder mehrfach durch Halogen, $C_1$-$C_4$-Alkyl oder $C_1$-$C_4$-Halogenalkyl substituiertes Aryl steht,
$R^4$ für Hydroxy, $C_1$-$C_4$-Alkyl oder $C_1$-$C_4$-Alkoxy steht,

**dadurch gekennzeichnet, dass** Bernsteinsäuremonoamide der Formel (VI)

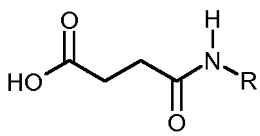

(VI)

(VI)

in welcher R für $R^1$ oder $R^2$ steht,
mit Thionylchlorid zu einer Reaktionsmischung umgesetzt werden, in einer zweiten Stufe (2) die entstandene Reaktionsmischung erhitzt wird und anschließend die Umsetzung der Reaktionsmischung zu den Dithiin-tetracarboximiden im dritten Schritt (3) erfolgt, wobei mindestens Schritt (2) kontinuierlich durchgeführt wird.

2. Verfahren gemäß Anspruch 1, **dadurch gekennzeichnet, dass** die Gesamtmenge an Thionylchlorid (z) zwischen 2,5 und 20 Mol pro Mol Bernsteinsäuremonoamid der Formel (VI) beträgt, wobei z durch die Beziehung

$$z = x + y$$

definiert wird und

z für die Gesamtmenge an Thionylchlorid (Mol Thionylchlorid pro Mol Bernsteinsäureamid der allgemeinen Formel (VI)) in den beiden ersten Schritten (1) und (2),
x für die Menge Thionylchlorid in Schritt (1) und
y für die in Schritt (2) zusätzlich eingesetzte Menge Thionylchlorid steht.

3. Verfahren gemäß Anspruch 2, **dadurch gekennzeichnet, dass** der Wert z zwischen 2,5 und 14 Mol pro Mol Bernsteinsäuremonoamid der Formel (VI) beträgt

4. Verfahren gemäß Anspruch 2, **dadurch gekennzeichnet, dass** der Wert z zwischen 2,5 und 9 Mol pro Mol Bernsteinsäuremonoamid der Formel (VI) beträgt.

5. Verfahren gemäß Anspruch 2, **dadurch gekennzeichnet, dass** die Menge an Thionylchlorid x in Schritt (1) zwischen 1 und 20 Mol pro Mol Bernsteinsäuremonoamid der Formel (VI) beträgt.

6. Verfahren gemäß Anspruch 1, **dadurch gekennzeichnet, dass** nach Abklingen der Gasentwicklung in Schritt (2) die Umsetzung der Reaktionsmischung zu den Dithiin-tetracarboximiden im dritten Schritt (3) des Verfahrens erfolgt.

7. Verfahren gemäß Anspruch 6, **dadurch gekennzeichnet, dass** das erhaltene Zwischenprodukt in einem Verdünnungsmittel gelöst, mit Wasser versetzt und durch Erwärmen in diesem Gemisch in die Dithiin-tetracarboximide der Formel (I) überführt wird.

8. Verfahren gemäß Anspruch 1, **dadurch gekennzeichnet, dass** Schritt (1) kontinuierlich durchgeführt wird.

9. Verfahren gemäß Anspruch 1, **dadurch gekennzeichnet, dass** in Schritt (3) ein organisches Lösungsmittel eingesetzt wird, welches wenigstens teilweise mit Wasser mischbar ist.

10. Verfahren gemäß Anspruch 1, **dadurch gekennzeichnet, dass** in Schritt (3) als Lösungsmittel Wasser, Dimethylsulfoxid, Sulfolan, Alkohole wie Methanol, Ethanol, Propanol, Isopropanol, 1- Butanol, 2-Butanol, Isobutanol, Tertiärbutanol, 1-Pentanol, Cyclopentanol, Cyclohexanol, Ethylenglykol, Ethylenglkyol-monomethylether, Kohlenwasserstoffe wie Hexan, Heptan, Cyclohexan, Methylcyclohexan, Toluol, Xylole, Mesitylen, Chlorbenzol, Dichlorbenzol, Nitrobenzol, Ester wie Essigsäuremethylester, Essigsäureethylester, Amide wie Formamid, N,N-Dimethylformamid; N,N-Dimethylacetamid, N-Methylpyrrolidon, Ether wie Methyl-tert-butylether, Tetrahydrofuran, 1,4-Dioxan, Nitrile wie Acetonitril, Propionitril, Butyronitril, Benzonitril, Ketone wie Aceton, Methyl-ethylketon, Methyl-isobutylketon, Pinakolon, Carbonsäuren wie Ameisensäure, Essigsäure, Propionsäure oder Gemische dieser Verdünnungsmittel einsetzt werden.

11. Verfahren gemäß Anspruch 1, **dadurch gekennzeichnet, dass** Schritt (3) bei einer Reaktionstemperatur zwischen 40°C und 120°C durchgeführt wird.

**Claims**

1. Process for preparing dithiinetetracarboximides of the general formula (I)

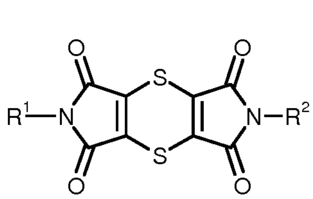

(I)

in which

$R^1$ and $R^2$ are the same or different and are each hydrogen, optionally mono- or poly-halogen-, $-OR^3$-, $-COR^4$-substituted $C_1$-$C_8$-alkyl, optionally mono- or poly-halogen-, -$C_1$-$C_4$-alkyl- or -$C_1$-$C_4$-haloalkyl-substituted $C_3$-$C_7$-cycloalkyl, in each case optionally mono- or poly-halogen-, -$C_1$-$C_4$-alkyl-, -$C_1$-$C_4$-haloalkyl-, $-COR^4$- or -sulphonylamino-substituted aryl or aryl-($C_1$-$C_4$-alkyl),
$R^3$ is hydrogen, $C_1$-$C_4$-alkyl, $C_1$-$C_4$-alkylcarbonyl or optionally mono- or poly-halogen-, -$C_1$-$C_4$-alkyl- or

$-C_1-C_4$-haloalkyl-substituted aryl,
$R^4$ is hydroxyl, $C_1-C_4$-alkyl or $C_1-C_4$-alkoxy,

**characterized in that** succinic monoamides of the formula (VI)

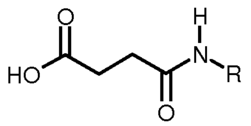

(VI)

(VI)

in which R is $R^1$ or $R^2$
are reacted with thionyl chloride to give a reaction mixture, the reaction mixture formed is heated in a second stage (2), and then the reaction mixture is converted to the dithiinetetracarboximides in the third step (3), with continuous performance of at least step (2).

2. Process according to Claim 1, **characterized in that** the total amount of thionyl chloride (z) is between 2.5 and 20 mol per mole of succinic monoamide of the formula (VI), where z is defined by the relationship

$$z = x + y$$

and

z is the total amount of thionyl chloride (mol of thionyl chloride per mole of succinamide of the general formula (VI)) in the first two steps (1) and (2),
x is the amount of thionyl chloride in step (1) and
y is the amount of thionyl chloride additionally used in step (2).

3. Process according to Claim 2, **characterized in that** the value z is between 2.5 and 14 mol per mole of succinic monoamide of the formula (VI).

4. Process according to Claim 2, **characterized in that** the value z is between 2.5 and 9 mol per mole of succinic monoamide of the formula (VI).

5. Process according to Claim 2, **characterized in that** the amount of thionyl chloride x in step (1) is between 1 and 20 mol per mole of succinic monoamide of the formula (VI).

6. Process according to Claim 1, **characterized in that** abatement of the evolution of gas in step (2) is followed by the conversion of the reaction mixture to the dithiinetetracarboximides in the third step (3) of the process.

7. Process according to Claim 6, **characterized in that** the resulting intermediate is dissolved in a diluent, admixed with water and converted to the dithiinetetracarboximides of the formula (I) by heating in this mixture.

8. Process according to Claim 1, **characterized in that** step (1) is performed continuously.

9. Process according to Claim 1, **characterized in that** an organic solvent at least partly miscible with water is used in step (3).

10. Process according to Claim 1, **characterized in that** the solvents used in step (3) are water, dimethyl sulphoxide, sulpholane, alcohols such as methanol, ethanol, propanol, isopropanol, 1-butanol, 2-butanol, isobutanol, tert-butanol, 1-pentanol, cyclopentanol, cyclohexanol, ethylene glycol, ethylene glycol monomethyl ether, hydrocarbons such as hexane, heptane, cyclohexane, methylcyclohexane, toluene, xylenes, mesitylene, chlorobenzene, dichlorobenzene, nitrobenzene, esters such as methyl acetate, ethyl acetate, amides such as formamide, N,N-dimethylformamide; N,N-dimethylacetamide, N-methylpyrrolidone, ethers such as methyl tert-butyl ether, tetrahydrofuran, 1,4-dioxane, nitriles such as acetonitrile, propionitrile, butyronitrile, benzonitrile, ketones such as acetone, methyl ethyl ketone, methyl isobutyl ketone, pinacolone, carboxylic acids such as formic acid, acetic acid, propionic acid, or mixtures of

these diluents.

**11.** Process according to Claim 1, **characterized in that** step (3) is performed at a reaction temperature between 40°C and 120°C.

## Revendications

**1.** Procédé pour la préparation de dithiine-tétracarboximides de formule générale (I)

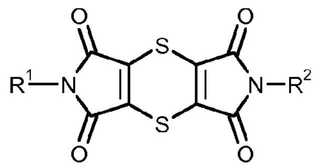

(I)

dans laquelle

$R^1$ et $R^2$ sont identiques ou différents et représentent hydrogène, $C_1$-$C_8$-alkyle le cas échéant monosubstitué ou polysubstitué par halogène, -$OR^3$, -$COR^4$ ; $C_3$-$C_7$-cycloalkyle le cas échéant monosubstitué ou polysubstitué par halogène, $C_1$-$C_4$-alkyle ou $C_1$-$C_4$-halogénoalkyle ; aryle ou aryl-($C_1$-$C_4$-alkyle) à chaque fois le cas échéant monosubstitué ou polysubstitué par halogène, $C_1$-$C_4$-alkyle, $C_1$-$C_4$-halogénoalkyle, -$COR^4$ ou sulfonylamino,
$R^3$ représente hydrogène ; $C_1$-$C_4$-alkyle ; $C_1$-$C_4$-alkylcarbonyle ; ou aryle le cas échéant monosubstitué ou polysubstitué par halogène, $C_1$-$C_4$-alkyle ou $C_1$-$C_4$-halogénoalkyle,
$R^4$ représente hydroxy, $C_1$-$C_4$-alkyle ou $C_1$-$C_4$-alcoxy,

**caractérisé en ce qu'**on transforme des monoamides de l'acide succinique de formule (VI)

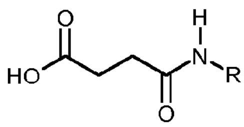

(VI)

(VI)

dans laquelle R représente $R^1$ ou $R^2$,
avec du chlorure de thionyle en un mélange réactionnel, dans une deuxième étape (2), on chauffe le mélange réactionnel formé et la transformation du mélange réactionnel en dithiine-tétracarboximides a ensuite lieu dans une troisième étape (3), au moins l'étape (2) étant réalisée en continu.

**2.** Procédé selon la revendication 1, **caractérisé en ce que** la quantité totale de chlorure de thionyle (z) est située entre 2,5 et 20 moles par mole de monoamide de l'acide succinique de formule (VI), z étant défini par la relation

$$z = x + y$$

et

z représentant la quantité totale de chlorure de thionyle (moles de chlorure de thionyle par mole d'amide de l'acide succinique de formule générale (VI)) dans les deux premières étapes (1) et (2),
x représentant la quantité de chlorure de thionyle dans l'étape (1) et
y représentant la quantité de chlorure de thionyle ajoutée en plus dans l'étape (2).

**3.** Procédé selon la revendication 2, **caractérisé en ce que** la valeur z est située entre 2,5 et 14 moles par mole d'amide de l'acide succinique de formule (VI).

**4.** Procédé selon la revendication 2, **caractérisé en ce que** la valeur z est située entre 2,5 et 9 moles par mole d'amide

de l'acide succinique de formule (VI).

5. Procédé selon la revendication 2, **caractérisé en ce que** la quantité de chlorure de thionyle x dans l'étape (1) est située entre 1 et 20 moles par mole de monoamide de l'acide succinique de formule (VI).

6. Procédé selon la revendication 1, **caractérisé en ce qu'**après l'affaiblissement du dégagement gazeux dans l'étape (2), la transformation du mélange réactionnel en dithiine-tétracarboximides a lieu dans la troisième étape (3) du procédé.

7. Procédé selon la revendication 6, **caractérisé en ce que** le produit intermédiaire obtenu est dissous dans un diluant, additionné d'eau et transformé par chauffage dans ce mélange en dithiine-tétracarboximides de formule (I).

8. Procédé selon la revendication 1, **caractérisé en ce que** l'étape (1) est réalisée en continu.

9. Procédé selon la revendication 1, **caractérisé en ce qu'**un solvant organique, qui est au moins partiellement miscible à l'eau, est utilisé dans l'étape (3).

10. Procédé selon la revendication 1, **caractérisé en ce qu'**on utilise, dans l'étape (3), comme solvant, de l'eau, du diméthylsulfoxyde, du sulfolane, des alcools tels que le méthanol, l'éthanol, le propanol, l'isopropanol, le 1-butanol, le 2-butanol, l'isobutanol, le tert-butanol, le 1-pentanol, le cyclopentanol, le cyclohexanol, l'éthylèneglycol, l'éthylèneglycolmonométhyléther, des hydrocarbures tels que l'hexane, l'heptane, le cyclohexane, le méthylcyclohexane, le toluène, les xylènes, le mésitylène, le chlorobenzène, le dichlorobenzène, le nitrobenzène, des esters tels que l'ester méthylique de l'acide acétique, l'ester éthylique de l'acide acétique, des amides tels que le formamide, le N,N-diméthylformamide, le N,N-diméthylacétamide, la N-méthylpyrrolidone, des éthers tels que le méthyl-tert-butyléther, le tétrahydrofuranne, le 1,4-dioxane, des nitriles tels que l'acétonitrile, le propionitrile, le butyronitrile, le benzonitrile, des cétones telles que l'acétone, la méthyléthylcétone, la méthylisobutylcétone, la pinacolone, des acides carboxyliques tels que l'acide formique, l'acide acétique, l'acide propionique ou des mélanges de ces diluants.

11. Procédé selon la revendication 1, **caractérisé en ce que** l'étape (3) est réalisée à une température de réaction entre 40°C et 120°C.

Figur 1

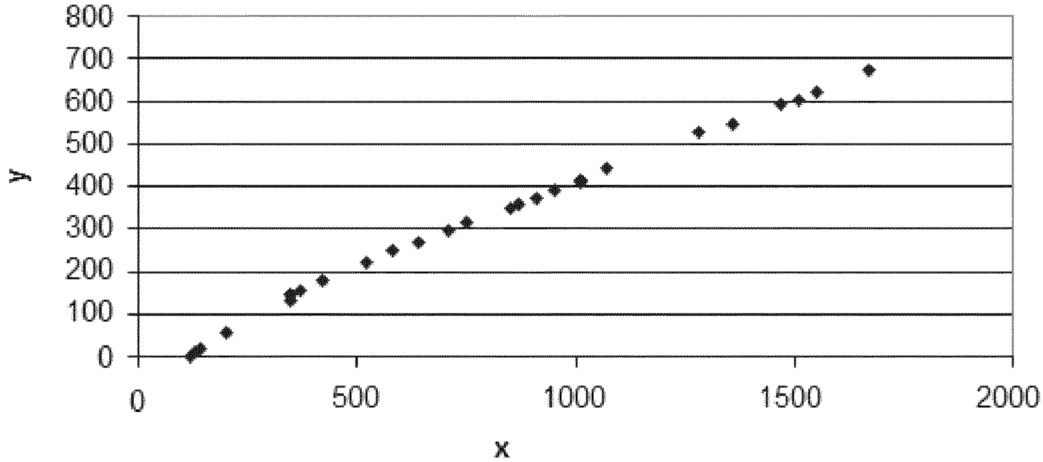

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3364229 A
- JP 10251265 A
- PL 143804 B
- WO 2011128263 A1
- WO 2011128263 A


### In der Beschreibung aufgeführte Nicht-Patentliteratur


- *Il Farmaco,* 2005, vol. 60, 944-947
- *Chem. Ber.,* 1967, vol. 100, 1559-1570
- *J. Heterocycl. Chem.,* 1988, vol. 25, 901-906
- *Synthetic Communications,* 2006, vol. 36, 3591-3597
- *J. Heterocycl. Chem.,* 2010, vol. 47, 188-193